# EUROPEAN PATENT SPECIFICATION

(11) **EP 2 446 251 B1**
(45) Date of publication and mention of the grant of the patent: **05.12.2018**
(21) Application number: 10792422.7
(22) Date of filing: 24.06.2010
(51) Int. Cl.: G03H 1/00, G03H 1/04, G03H 1/08, G06K 9/00, G01N 21/45

(54) **ANALYSIS OF OVA OR EMBRYOS WITH DIGITAL HOLOGRAPHIC IMAGING**
ANALYSE VON EIZELLEN UND EMBRYOS MIT DIGITALER HOLOGRAFISCHER ABBILDUNG
ANALYSE D'OVULES OU D'EMBRYONS PAR IMAGERIE HOLOGRAPHIQUE NUMÉRIQUE

(30) Priority: 25.06.2009 SE 0950491; 25.06.2009 US 220350 P
(43) Date of publication of application: 02.05.2012
(73) Proprietor: Phase Holographic Imaging Phi AB, 223 63 Lund (SE)
(72) Inventor: SEBESTA, Mikael, S-247 50 Dalby (SE); PERSSON, Johan, S-216 11 Limhamn (SE); GISSELSSON, Lennart, S-227 63 Lund (SE); MÖLDER, Anna, S-275 64 Blentarp (SE); LÅNGBERG, Anders, S-231 95 Trelleborg (SE)
(74) Representative: AWA Sweden AB
(86) International application number: PCT/SE2010/050726
(87) International publication number: WO 2010/151221

(56) References cited:
- DE-A1-102005 036 326
- DE-B3-102005 030 899
- US-A1- 2005 036 181
- US-A1- 2007 216 906
- US-A1- 2008 032 325
- US-B2- 7 486 406
- MANN CHRISTOPHER J ET AL: "Movies of cellular and sub-cellular motion by digital holographic microscopy", BIOMEDICAL ENGINEERING ONLINE, BIOMED CENTRAL LTD, LONDON, GB, vol. 5, no. 1, 23 March 2006 (2006-03-23), page 21, XP021018046, ISSN: 1475-925X, DOI: 10.1186/1475-925X-5-21
- GASS ET AL: "Phase imaging without 2 Pi ambiguity by multiwavelength digital holography", OPTICS LETTERS, vol. 28, no. 13, 1 July 2003 (2003-07-01), pages 1141-1143, XP55039771,
- MANN C. J. ET AL: 'Movies of cellular and sub-cellular motion by digital holographic microscopy' BIOMEDICAL ENGINEERING ONLINE vol. 5, no. 21, 23 March 2006, LONDON, GB, pages 1 - 10, XP021018046
- KEMPER B. ET AL: 'Modular digital holographic microscopy system for marker free quantitative phase contrast imaging of living cells' PROCEEDINGS OF THE SPIE - THE INTERNATIONAL SOCIETY FOR OPTICAL ENGINEERING vol. 6191, 2006, pages 1 - 8, XP003027053
- KEMPER B. ET AL: '3D tracking and multi-wavelength techniques for digital holographic microscopy based cell analysis' PROGRESS IN BIOMEDICAL OPTICS AND IMAGING - PROCEEDINGS OF SPIE - ADVANCED MICROSCOPY TECHNIQUES vol. 7367, 2009, USA, pages 1 - 8, XP009141099
- EMERY Y. ET AL: 'DHM (Digital Holography Microscope) for imaging cells' JOURNAL OF PHYSICS: CONFERENCE SERIES vol. 61, 2007, pages 1317 - 1321, XP020124658
- KEMPER B. ET AL: 'Digital holographic microscopy for live cell applications and technical inspection' APPLIED OPTICS vol. 47, no. 4, 01 February 2008, pages A52 - A61, XP007904218
- WENJING ZHOU ET AL: 'Phase reconstruction of live human embryonic kidney 293 cells based on two off-axis holograms' PROC. OF SPIE vol. 7375, 2008, pages 1 - 8, XP003027054
- KEMMLER M. ET AL.: 'Noninvasive time-dependent cytometry monitoring by digital holography' JOURNAL OF BIOMEDICAL OPTICS vol. 12, no. 6, 2007, pages 1 - 10, XP003027055
- PALMSTIERNA M ET AL: "Zona pellucida thickness variation and occurrence of visible mononucleated blastomers in preembryos are associated with a high pregnancy rate in IVF treatment", JOURNAL OF ASSISTED REPRODUCTION AND GENETICS, PLENUM PUBLISHING, US, vol. 15, no. 2, 1 February 1998 (1998-02-01), pages 70-75, XP009090407, ISSN: 1058-0468

## Description

### Technical Field

The present invention relates to use of a method for analyzing a sample comprising at least one ovum or embryo.

### Technical Background

In vitro fertilization (IVF) has been carried out since the late 1970's and in order to increase the probability for a successful pregnancy and a healthy baby the demand for more accurate predictability of ovum and embryo quality has increased ever since. The present standard implantation technique using two or three embryos results in a risk for twin pregnancies, which are associated with a risk for congenital conditions. Further the implantation rate today is approximately 40%. Current techniques often rely on subjective judgment based on experience with the help of some basic parameters. The ovum and embryos are usually inspected ocularly through a phase contrast microscope and thus the quality of the inspection is strongly dependent on the skill of the person conducting the ocular inspection. The current available quantitative methods are often very technically and environmentally demanding or only provide rudimentary data through software analysis of a phase contrast image. In case of a human embryo, the embryo is usually studied after 2 and 5 days after fertilization.

In the prediction of ovum or embryo viability it is of most importance that the used method does not affect the cells. This is why different kind of dyes and fluorescence markers cannot be used. Other techniques involving high power light sources are also ruled out. The absolute majority of all ovum and embryo classification are made with phase contrast microscopy or a variant of phase contrast microscopy, e.g. differential interference contrast microscopy (DIC).

When studying an ovum or embryo by means of a phase contrast microscope, the person conducting the ocular inspection must virtually continuously manually set the focal plane in order to study different parts of the ovum or embryo, since the focal depth, i.e. the depth within which a sharp image is visible is smaller than the depth of a cell.

A method for counting cells in a sample of living tissue, such as an embryo, is disclosed in US 2008/0032325. The number of cells are counted by obtaining a first and a second image of a cell cluster, fitting an ellipse to the boundary of a cell in the second image and by means thereof produce an ellipsoidal model cell, subtracting the model cell from the first image to obtain a subtracted image and thereafter likewise subtract each cell until no cells are left in the subtracted image. The number of cells corresponds to the number of subtractions. Optical quadrature microscopy (OQM), polarization interferometry, digital holography, Fourier phase microscopy, Hilbert phase microscopy, quantitative phase microscopy, or diffraction phase microscopy are said to be possible methods for obtaining the first image, and OQM is preferred. Differential interference contrast microscopy (DIC), Hoffman interference contrast microscopy and brightfield microscopy are disclosed as possible methods for obtaining the second image, and DIC is preferred. In this technique both a first imaging modality, e.g. OQM, and a second imaging modality, e.g. a differential interference contrast microscopy, are required. Thus, two microscopy methods are required. The OQM microscope includes a mach-zender interferometer and a full field imaging optical set-up. A phase image is created by interpreting interferograms from four different cameras where the reference light has travelled through a 1/4-wave plate to create a 90 degree phase shift of the orthogonal P-plane and S-plane polarization. Polarizing beam splitters are then used to separate the signal of the two polarization directions, which then are detected on two separate cameras. Two additional cameras are also used to detect the conjugate intensities of the signal. The functionality of the lenses in the microscopes used in US 2008/0032325 is to create an image, i.e. a representation of the studied object.

Furthermore, Mann Christopher J et al. in "Movies of cellular and sub-cellular motion by digital holographic microscopy" , BioMedical Engineering OnLine 2006. 5:21, published March 23, 2006, discloses the use of a modified Mach-Zender off-axis setup in transmission to record and reconstruct a number of holographic amplitude and phase images of cellular and sub-cellular features. The cells evaluated are mouse embryo fibroblast cell.

Moreover, Gass et al in "Phase imaging without 2n ambiguity by multiwavelength digital holography", OPTICS LETTERS/ Vol. 28, No. 13 / July 1, 2003, discloses a phase-imaging method consisting of the generation and combination of two phase maps in a digital holography system by use of two separate wavelengths.

Further, Palmstierna et al is in "Zona Pellucida Thickness Variation and Occurrence of Visible Mononucleated Blastomeres in Preembryos Are Associated with a High Pregnancy Rate in IVF Treatment", Journal of Assisted Reproduction and Genetics, Vol. 15, No. 2, 1998, evaluating the ability of six morphological criteria; embryo development rate, fragmentation, regularity of blastomere shape, equality of blastomere size, zona pellucida thickness variation (ZPTV), and visible mononucleated blastomeres (VMBs) to predict pregnancy in IVF treatment cycles.

Further, US 2005/0036181 discloses an apparatus and method for digital holographic imaging. In this document the information content of one hologram, or of a plurality of holograms, recorded with a sample in one medium, or in a plurality of medium, is used to reconstruct one image of the sample, or a plurality of images of the sample, that describe quantitatively one property of the sample, or a plurality of properties of the sample.

### Summary of the Invention

One object of the present invention is to be able to analyze and classify the quality, such as the viability, of an ovum or embryo without the need for a judgment of a person. The accuracy of the judgment is strongly dependent on the skill and experience of the person conducting the ocular inspection. One further object of the present invention is to provide a quantitative method, which is possible to perform with less optical demanding, less sensitive as well as less expensive equipment than the existing ones.

According to a first aspect, the above and further objects are solved by use of a method for analyzing a sample comprising at least one ovum or embryo, the method being solely based on digital holographic imaging, the method comprising the following steps:
a) creating at least one object beam and at least one reference beam of light, where said at least one object beam and said at least one reference beam are mutually coherent;
b) exposing said sample to said at least one object beam;
c) superimposing said at least one object beam that has passed through said sample with said at least one reference beam and thereby creating an interference pattern;
d) detecting said interference pattern, called hologram;
e) reconstructing phase and/or amplitude information of object wavefront from said interference pattern; and
f) constructing at least one ovum or embryo analysis image and determining at least one ovum or embryo quality showing parameter from said phase and/or amplitude information, wherein the at least one ovum or embryo quality showing parameter embodies at least one of the parameters chosen from the group consisting of the radial thickness of the zona pellucida of the at least one ovum or embryo; the optical density of the zona pellucida of the at least one ovum or embryo; the dry mass, morphology or area of the at least one ovum or at least one cell inside the at least one embryo; and the dry mass, morphology or area of at least one polar body.

An ovum or embryo quality showing parameter determined according to the method above may be used in valuation of the quality of an ovum or embryo.

The method of the present invention enables analyses and classification of the quality, such as the viability, of an ovum or embryo without the need for a judgment of a person. The accuracy of a judgment is strongly dependent on the skill and experience of the person conducting the ocular inspection and this is avoided by the method according to the present invention. The method may also be used as a complement to an ocular inspection.

By the method according to the present invention advanced information about ova or embryos may be obtained by relatively inexpensive equipment using only one imaging technology. In particular, the method of the present invention enables analysis using inexpensive equipment compared with the expensive and complicated equipment involved in OQM, which includes four optical cameras. In addition, the equipment that is necessary for the conduction of the method according to the present invention is stable and not particularly sensitive to extraneous disturbances, in particular compared with the equipment involved in OQM. In addition, the method of the present invention only has to involve one camera in order to carry out the analysis of the ovum or embryo.

By the method of the present invention it is also possible to determine several ovum or embryo quality showing parameters and base the classification of the ovum or embryo on these several parameters.

Digital holography is a very lenient method, which enables studies of living cells without the need for markers or stains and enables quantification of the studied objects. Digital holography enables studies without having to mark or stain the objects, which is time consuming and costly. Thus, digital holography enables the study of living cells and the development of cells, such as cell growth. Digital holography is at least as lenient as phase contrast microscopy.

Phase contrast microscopy is a technique where variations in the refractive index and thickness of the object give rise to an increased contrast in the images. In digital holography these variations give rise to a phase shift of the incident light which can be quantitatively measured. This gives the possibility to measure the optical path length in every pixel in the images and hence gives an accurate tool in ovum or embryo classification.

Digital holography does not include image formation optics as it only registers the interference between the reference beam of light and the object beam of light. Basically, the primary purpose of the lenses used in a digital holographic microscope is not to create an image of the studied object, instead the lenses are used to gather light. Thereafter, by means of reconstruction algorithms conducted by a computer, an image of the studied object is obtained. The algorithm uses the interference pattern recorded to reconstruct the complex optical wave field at a chosen observation plane or volume. The method requires a minimum of optical components and incorporates numerical focalization and compensation for aberration as it can be considered a digitized imaging system. This implies that there are not particularly high requirements on the optical accuracy of the microscope, such as the lenses.

When using digital holography, it is not necessary to set the focal plane. Contrary, information is collected and thereafter it is by reconstruction algorithms possible to obtain images in any wished observation plane or volume.

Further features and advantages of the invention are disclosed in more detail in the following.

### Brief description of the drawings

Fig 1 is a sketch of two embryos showing the first sign of a successful fertilization.
Fig 2 is a sketch of a zona pellucida surrounding a polar body and a single embryonic cell.
Fig 3 is a sketch of two embryos comprising several cells.
Fig 4 is a schematic illustration of an apparatus according to an example not covered by the claims of the present invention.

### Detailed description of the invention

The present invention will be described in more detail below. As used herein, an ovum or embryo quality showing parameter means any parameter giving information about the quality of at least one ovum or embryo. In particular, an ovum or embryo quality showing parameter gives information about the quality of at least one ovum or embryo relevant for the use of said at least one ovum or embryo in fertilization treatment. The ovum or embryo quality showing parameter may be chosen from, but are not limited to, the radial thickness of the zona pellucida of the at least one ovum or embryo; the optical density of the zona pellucida of the at least one ovum or embryo; the dry mass, morphology or area of the at least one ovum or at least one cell inside the at least one embryo; the dry mass, morphology or area of at least one polar body; comparison of the dry mass, morphology or area of one ovum to at least one other; comparison of the dry mass, morphology or area of one cell inside the at least one embryo to at least one other; determination of the number of ova in the sample; determination of the number of cells inside the at least one embryo; determination of the number of pronuclei inside the at least one ovum or embryo; determination of the total dry mass of at least one defined space inside the at least one embryo; comparison of the total dry mass of one defined space inside the at least one embryo to at least one other; the level of fragmentation; the degree of synchronization; the volume of the at least one ovum or embryo or the volume of at least one cell inside the at least one embryo; the refractive index of at least one defined space inside the at least one ovum or embryo; and a combination of these.

In one embodiment of the method according to the present invention, two or more ovum or embryo quality showing parameters are determined and these parameters are combined into one or a few parameters representing the quality of an ovum or embryo.

In one embodiment of the method according to the present invention, each of the determined ovum or embryo quality showing parameter may be represented by a value depending on the quality of the ovum or embryo in respect of this specific parameter, e.g. each determined ovum or embryo quality showing parameter may be represented by a value on a scale, for example 1-10. The values representing the determined ovum or embryo quality showing parameters may be combined into one specific value representing the quality of an ovum or embryo. Preferably, a factor representing the importance of each of the ovum or embryo quality showing parameters are used when combining these parameters. This combined value will give very direct information regarding the quality of each ovum or embryo and will place the analyzed ova or embryos in order of precedence.

In one embodiment of the method according to the present invention, the mutually coherent at least one object beam and at least one reference beam of light are created by dividing a light beam originating from a coherent light source into two beams e.g. by means of a beam splitter. The light beam originating from a coherent light source may be a laser beam. The laser beam may originate from any kind of laser source, such as a He-Ne laser or a diode laser. Preferably a diode laser is used.

The object beam and the reference beam are mutually coherent, which implies that they have the same frequency and exhibit a constant phase relationship during the course of time.

The object beam is passed through the at least one ovum or embryo. The reference beam is left unaffected by the at least one ovum or embryo, since the reference beam is guided another path than the object beam, e.g. by means of beam splitters, mirrors and/or fibre optics.

The object beam has a known wavefront before passing through the sample comprising at least one ovum or embryo. When the object beam passes through the at least one ovum or embryo, the ovum (ova) or embryo(s) substantially does (do) not absorb any light, but the light that travels through the ovum (ova) or embryo(s) will experience a difference in the optical path length compared to the surrounding medium. The wavefront that emerges from the ovum (ova) or embryo(s), the object wavefront, will thus be phase shifted. Naturally, also the reference beam has a known wavefront. The optical path length is defined as the physical/geometrical thickness multiplied with the refractive index.

In one embodiment of the method according to the present invention, the superimposing of the at least one object beam that has passed through the sample comprising at least one ovum or embryo and the at least one reference beam is achieved by bringing the two beams together e.g. by means of another beam splitter. This superimposition gives rise to an interference pattern, which for example includes information about the object wavefront that is affected by the at least one ovum or embryo.

In one embodiment of the method according to the present invention, the interference pattern is detected by means of a digital sensor, such as a CCD or a CMOS. The detected interference pattern is called a hologram.

In order to superimpose the at least one object beam that has passed through the sample comprising at least one ovum or embryo and the at least one reference beam and thereby creating an interference pattern and to detect the interference pattern for example a Fourier setup or a Fresnel setup may be used. Preferably a Fresnel setup is used. The difference between a Fourier setup and a Fresnel setup may be described as a difference in the optical configuration implying that a certain condition is fulfilled which makes an approximation, e.g. a Fresnel approximation, applicable in the reconstruction algorithm. This approximation simplifies the process of image reconstruction. In case of a Fresnel setup, the condition is that the distance between the object and the sensor is large compared to the size of the object and the size of the sensor. This is achieved by use of a microscope objective that collects the scattered light from the object and directs it to the sensor in an almost parallel light beam. This creates a virtual object that is positioned far away from the sensor.

From the detected interference pattern phase and/or amplitude information of the object wavefront is reconstructed. The reconstruction is carried out by means of any common numerical reconstruction process such as Fourier transform reconstruction or convolution reconstruction. The amplitude information may be used to set the focal plane of interest. The reconstructed information may for example be used to obtain an image in two dimensions or a 3D representation of the studied at least one ovum or embryo.

The obtained image is further used in image processing conducted by a computer in order to determine useful information about the studied object, such as parameters relevant for the quality of the studied ova or embryos.

Several obtained images may also be further used in image processing in order to determine additional useful information about the studied object, such as parameters relevant for the quality of the studied ova or embryos.

Such image processing may involve segmentation algorithms, wherein predefined shapes are located. These located predefined shapes may be further analyzed, e.g. regarding their size (area or volume), dry mass, number etc. One type of segmentation algorithm that may be used is a watershed segmentation.

As an alternative for creating one object beam and one reference beam, in-line digital holography may be used. It is obvious for a person skilled in the art how to modify the method of the present invention in order to use in-line digital holography, when studying this specification.

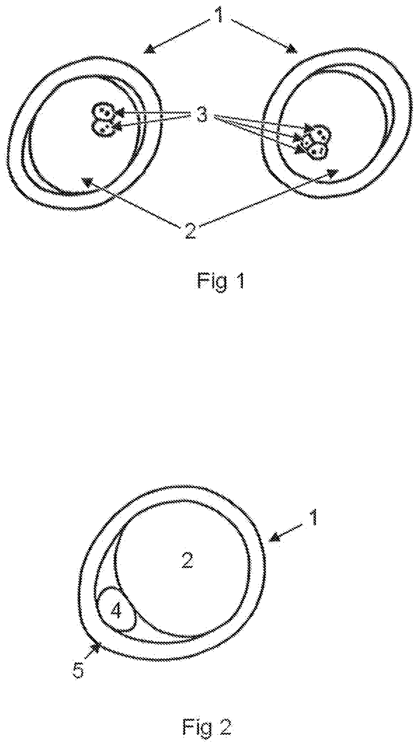

An ovum (oocyte, egg) normally consists of a single cell. A mature human oocyte is approximately 100-120 µm making it one of the largest cells in the body. The oocyte is essentially circular. As used herein an embryo is a fertilized oocyte. During the first few days after fertilization a number of events occur that strongly indicates the quality of the embryo. The oocyte contains a single pronucleus and after fertilization the embryo contains two pronuclei visible as small circular shapes within the single cell embryo, as can be seen in figure 1. The single cell embryo is referred to as a zygote. The first visible stage of fertilization is the presence of a second pronucleus. As this has occurred the embryo divide for the first time, yielding 2 cells. The cells go through further mitosis (cell division) forming 4, 8 and 16 cell stages *etc* under the assumption of synchronized cell division. An embryo comprising more than one cell is referred to as a morula. After several cell divisions a blastula having a spherical layer of several cells surrounding a central fluid-filled cavity called the blastocoel is formed. The development of the blastula, during which the volume of the blastocoel increases, is vital. The blastula transforms into a blastocyst having an inner cell mass, named embryoblast, an outer layer of cells, named trophoblast, which later forms the placenta, and a blastocyst cavity located inside the trophoblast. The amount and density of the cells in the inner cell mass are vital. Also the size (i.e. area or volume) and density of trophoblasts in the outer layer are vital. In the case of a human embryo, the blastocyst is formed around day 5 after fertilization.

The proportion between the embryoblast, the trophoblast and the blastocyst cavity gives information about the quality of the embryo in the blastocyst stage. Therefore, in order to evaluate the quality of the embryo, the three zones, i.e. the embryoblast, the trophoblast and the blastocyst cavity, may be compared with each other. It may be valuable to compare the dry mass, the area as well as the optical density of these three zones. Also the total dry mass, area and optical density may give information about the quality of the embryo.

By dry mass, as used herein, is meant the non water cellular substances, such as proteins, of a studied object or part of object. The optical path length is directly related to the dry mass. When a water based liquid is used as medium surrounding the object in the sample, the optical path length is a direct measure of the protein content, since the water in the cell corresponds to the medium.

In one embodiment of the method according to the present invention, the at least one ovum or embryo is at least one ovum.

In one embodiment of the method according to the present invention, the at least one ovum or embryo is at least one embryo.

In one embodiment of the method according to the present invention the at least one embryo comprises at least one cell.

In one embodiment of the method according to the present invention, the at least one ovum or embryo quality showing parameter embodies the dry mass, morphology or area of the at least one ovum or at least one cell inside the at least one embryo.

In one embodiment of the method according to the present invention, the at least one ovum or embryo is at least one embryo encapsulating more than one cell and an embryo quality showing parameter is determined by determining the total dry mass of at least one defined space inside the at least one embryo.

In one further embodiment of the method according to the present invention, the total dry mass of more than one defined space inside the at least one embryo is determined and the total dry mass of one space is compared to at least one other.

The ovum and embryo is surrounded by the halo-like zona pellucida, which is visible in figure 2. The zona pellucida is a multilayer glycoprotein coat surrounding the mammalians from the early oocyte to the early embryo. *In vivo,* the zona pellucida provides a vital component in preventing poly-spermia, i.e. fertilization with multiple sperm cells, as well as immunological responses (as the sperm may give rise to incompatibility with the host's immune system). *In vitro,* the zona pellucida serves an equally vital role of protecting the ovum and embryo from the mechanical stress of implantation. The radial thickness of the zona pellucida is therefore an important feature of the quality of an ovum or embryo. By the radial thickness of the zona pellucida is meant the radial distance from the inner boundary to the outer boundary of the zona pellucida. By studying zona pellucida by means of the method according to the present application it is possible to obtain a measurement of the radial thickness of zona pellucida throughout the entire circumference of the ovum or embryo. The radial thickness of the zona pellucida may be presented as average, maximum and/or minimum radial thickness as well as visualized as a graph representing the radial thickness of the zona pellucida along the circumference of the ovum or embryo. Thereby variations of the radial thickness of the zona pellucida may be obtained. The gradual thinning of the zona pellucida may also be observed.

The optical density of the zona pellucida is an important feature of the quality of an ovum or embryo. Also the optical density of the zona pellucida may be obtained by the method according to the present invention. As the radial thickness, also the optical density of the zona pellucida may be presented as average, maximum and/or minimum optical density as well as visualized as a graph representing the optical density of the zona pellucida along the circumference of the ovum or embryo. The optical density of different layers of the zona pellucida may also be vital. Also, possible cracks in the zona pellucida may be studied by the method according to the present invention.

The quality of an embryo may also be obtained by studying the development of the zona pellucida. Following sperm penetration, cortical granules, a special organelle in eggs, release their contents into the perivitelline space, a space between the oocyte and the zona pellucida in which polar bodies are located, in an event that is termed the cortical reaction. Cortical granules exudates alters the properties of the zona pellucida, which is known as zona reaction, and thus block polyspermic penetration. Changes in the zona architecture, which causes hardening of the zona during fertilization, is accompanied by changes in the secondary structure of the zona protein with a significant increase in the beta structure content. Thus, after fertilization the existing structures of the zona pellucida are rearranged and the quality of an embryo may be determined by studying the oocyte/- embryo before, during and after fertilization.

In one embodiment of the method according to the present invention, the at least one ovum or embryo quality showing parameter embodies at least one of the parameters chosen from the group consisting of the radial thickness of the zona pellucida of the at least one ovum or embryo and the optical density of the zona pellucida of the at least one ovum or embryo.

The polar bodies are cells with a small amount of cytoplasm formed after asymmetrical mitoses during meiosis. The polar bodies degenerate over time and may be fragmented. One polar body is extruded during the oocyte maturation process, prior to fertilization. The second polar body is extruded after the fertilization has taken place. One of the first signs of development is the presence of two polar bodies. n the single-cell state of the embryo the extrusion of the second polar body is often seen as an early sign of a possibly viable fertilization. The size (i.e. area or volume) of the polar body is related to the quality of the embryo with larger polar bodies being connected to lower rates of fertilization and embryos of worse quality. Fragmentation of the first polar body may be linked to the quality of an embryo. By measuring the size (i.e. area or volume) and amount (depending on time-point) of polar bodies it is possible to improve the rate at which fertilization occurs and good quality embryos may be formed.

In one embodiment of the method according to the present invention, the at least one ovum or embryo quality showing parameter embodies the dry mass, morphology or area of at least one polar body.

The pronucleus is the nucleus of the gamete, i.e. the sperm and ovum, prior to fusion fertilization. One of the very first signs of a successful fertilization is the presence of two pronuclei. No more than two should be present or else the embryo is of a low quality and should preferably be discarded. The quality of an embryo could thus be determined by counting the pronuclei after fertilization but prior to their fusion. Furthermore, nucleolar precursors are located within the pronucleus (figure 1) and yield information regarding the quality of the embryo. The arrangement of the nucleolar precursor bodies, the size (i.e. area or volume) of the pronuclei and the location of the nucleolar precursor bodies and the pronuclei in relation to each other are related to the quality of the embryo.

In one embodiment of the method according to the present invention, the at least one ovum or embryo quality showing parameter is determined by counting the number of pronuclei inside the at least one ovum or embryo.

The cells of an embryo go through mitosis (cell division) several times forming 2, 4, 8 and 16 cell stages *etc.* The number of cells and synchronization of mitosis of the blastomeres (initial cells in the embryo) serves as another basic and important parameter in deciding which embryos may be most suitable for implantation. The cells should substantially be in the same phase, i.e. cell division should occur in all cells at the same time, even though some variation always is expected in practice. Synchronization relates to cell division occurring simultaneously and cells dividing simultaneously are said to be synchronized. By counting the number of cells within an embryo by means of the method according to the present invention the degree of synchronization may be determined. The number of cells may be counted at several time-points prior to implantation in order to assure that the cell divisions are well-regulated.

In one embodiment of the method according to the present invention, the at least one ovum or embryo quality showing parameter embodies the level of fragmentation.

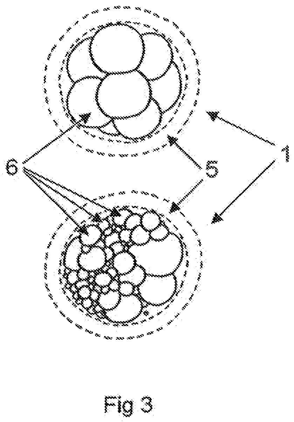

Fragmentation means the breaking apart of cells into smaller parts, which can be seen in figure 3. Presence of fragmentation in an ovum or embryo is a sign of that the ovum or embryo is of low quality, even though there quite often is some degree of fragmentation. Increased fragmentation is often linked to fewer cells in the embryo at observed time-points and may thus signal the lack of proper mitosis. In case of studying ova, increased fragmentation may be linked to several ova or ova fragments in the sample at observed time-points. By counting the number of ova in a sample or cells within an embryo by means of the method according to the present invention, the degree of fragmentation may be determined. Fragmentation may be due to genetic, metabolic defects or apoptotic (dying) cells or other unknown causes.

The number of ova or embryos may be determined by image processing using segmentation algorithms, wherein predefined shapes are located and the number of a specific type of shape corresponds to the number of ova or embryos. Preferably, watershed segmentation is used.

In one embodiment of the method according to the present invention, an ovum quality showing parameter is determined by counting the number of ova in the sample; or an embryo quality showing parameter is determined by counting the number of cells inside the at least one embryo.

In one embodiment of the method according to the present invention, the at least one ovum or embryo quality showing parameter embodies the degree of synchronization.

The total cytoplasmic volume (volume of cells or blastomeres not consisting of the nucleus) is constant up until early embryonic development and therefore each mitosis yields cells half the size of the previous. This has been proven true up until the 16 cell stage. By measuring the cell area or cell volume for each cell and compare them to each other enables the possibility to recognize embryos with unsynchronized cell cycles as well as fragmentation of ova or embryos.

In each cell stage, the dry mass of each cell should be the same in a embryo of high quality. Thus, by comparing the dry mass of each cell with the dry mass of the other cells of the embryo gives information about the quality of the embryo.

In one embodiment of the method according to the present invention, the sample comprises more than one ovum and an ovum quality showing parameter is determined by comparing the dry mass, morphology or area of one ovum to at least one other; or the at least one embryo encapsulates more than one cell and an embryo quality showing parameter is determined by comparing the dry mass, morphology or area of one cell inside the at least one embryo to at least one other.

To increase the information collected in the measurements of at least one ovum or embryo at least two wavelengths can be used. The refractive index is dependent on the wavelength. Therefore, by using two wavelengths both the physical/geometrical thickness and the refractive index can be measured in each pixel and not only the product of them. This means that the exact thickness of the object can be measured. Thus, not only the area, but also the volume of a cell, e.g. an ovum or a cell inside an embryo, or an embryo may be determined.

Also, different parts of a cell have different refractive index. For instance, the cell nucleus usually has a different refractive index than the cell body and by use of at least two wavelengths the cell nucleus can therefore be marked and measured separately in the digital holographic images.

Said at least two wavelengths can be achieved by using at least two different lasers or other light sources.

The use of at least two wavelengths facilitates the calculations related to the method of the present invention since less demanding algorithms may be used. When the interference pattern of the object is collected for two different wavelengths, the difference between the patterns can be interpreted as an interference pattern with a synthetic wavelength (beat wavelength) longer than the ones used to illuminate the sample. With this technique the modulo 2 pi optical path length information can be stretched longer than one time the wavelength of the incident beam without phase ambiguity. This is particularly interesting in the case with large objects with large phase shift such as ova or embryos. In addition, the use of at least two wavelengths yields images with less noise.

In one embodiment of the method according to the present invention, two or more wavelengths are used, the steps a) to e) are performed for each of the used wavelengths and the at least one ovum or embryo analysis image is constructed and the at least one ovum or embryo quality showing parameter is determined from the phase and/or amplitude information reconstructed using the two or more wavelengths.

In one embodiment of the method according to the present invention, the two or more wavelengths are achieved by two or more different lasers or other light sources.

When two or more wavelengths are used, the at least one ovum or embryo quality showing parameter is determined based on the information collected by using the two or more wavelengths. Also the at least one ovum or embryo analysis image is constructed based on the information collected by using the two or more wavelengths.

During early mitosis of the embryo there may be more or less than the expected single nucleus in each cell. Such irregular deviation in nuclei number is considered to yield ova or embryos of low quality. The number of nuclei may be determined from the standard ovum or embryo analysis image achieved by the method according to the present invention, but is more easily determined when at least two different wavelengths are used, since the different refractive index for the nuclei and the surrounding parts of the cell may be distinguished.

Even though the volume of a cell, such as an ovum, or an embryo may be determined by use of at least two wavelengths it is not possible to obtain the real three-dimensional morphology of a cell or an embryo since only the total physical thickness in each pixel is known. It is not known if the total physical thickness in a specific pixel is the total of one or several physical thicknesses, i.e. if there is some unoccupied space between two or more distances making up the total physical thickness. Three-dimensional images of a cell or an embryo may however be obtained based on assumptions relating to the normal morphology of the studied objects, i.e. cells, ova or embryos. These assumptions are facilitated if the cell or embryo is located on a flat surface. However, it is not possible to obtain real three-dimensional images based on the information obtained by using at least two wavelengths.

In one embodiment of the method according to the present invention, the at least one ovum or embryo quality showing parameter embodies the volume of the at least one ovum or embryo or the volume of at least one cell inside the at least one embryo.

In one embodiment of the method according to the present invention, the at least one ovum or embryo quality showing parameter embodies the refractive index of at least one defined space inside the at least one ovum or embryo.

Another way to increase the information collected is to take several images from different angles. If the ovum or embryo is rotated at a constant rotation speed and images are taken with specific intervals the images can be used in a tomographic reconstruction of the embryo. Alternatively, the ovum or embryo is rotated a specific angle at a time and after each rotation an image is taken. The object is rotated around an axis that not is parallel to the object beam. Preferably the object is rotated around an axis perpendicular to the object beam. The rotation of the ovum or embryo enables a real three-dimensional reconstruction of the ovum or embryo.

In one embodiment of the method according to the present invention, the sample comprising the at least one ovum or embryo is rotated and ovum or embryo analysis images are taken from different angles for the creation of a real three-dimensional reconstruction.

In one embodiment of the method according to the present invention, the ovum or embryo analysis images taken from different angles are combined by a tomographic algorithm for the creation of the real three-dimensional reconstruction.

When rotating the ovum or embryo it is possible to obtain the radial thickness and the optical density of the zona pellucida all around the substantially spherical ovum or embryo and not only along the circumference of the two dimensional projection of the ovum or embryo.

The ovum or embryo may be rotated e.g. mechanically, for example by a micropipette holding the ovum or embryo, or by means of optical tweezers or a micro-fluid device.

The two methods, i.e. using at least two wavelengths and taking images from different angles, can also be used in conjunction to maximize the information collected.

The above mentioned ovum or embryo quality showing parameters may rely on digital holographic images being captured at pre-determined time-points. With the correct culture conditions it would be possible to study the entire development through non-invasive time-lapse imaging. For example, images may be captured with a fixed interval, such as one hour or one day, and the captured images may be put together into a film displaying the development over time of the analyzed ovum or embryo. Also continuous capturing of images may be achieved with the method according to the present invention. Thus, it is possible to further determine the quality of ova or embryos. It is advantageous if the multi-nucleation occurs early and if the cleavage of the zygote occurs early. The time-point when multi-nucleation occurs and cleavage of the zygote occurs may be determined by studying an embryo at different time-points or continuously.

By capturing images at different time-points or continuously, the development of an ovum or embryo may be studied. It may for example be of significance to study the possible changes of the ovum, such as the changes of the zona pellucida, when fertilization occurs.

In one embodiment of the method according to the present invention, step b) to d) are repeated at different time-points or continuously in order to reconstruct phase and/or amplitude information according to step e) and construct ovum or embryo analysis images according to step f) captured at said time-points or continuously.

In one embodiment of the method, an ovum or embryo analysis image captured at one time-point is compared to an ovum or embryo analysis image captured at an earlier time-point. This is done in order to detect a change. For example, the number and/or the size of cells may be determined and compared in the analysis images captured at different times. In case a change has been detected, the time-point at which the change was detected may be stored. Additionally, the analysis image captured at the time-point at which a change was detected may be stored. Further, the comparison of the analysis images may be based on a comparison of at least one ovum or embryo quality showing parameter.

In one embodiment, at least the steps a)-d) are performed inside an incubator. In this way, the at least one ovum or embryo may conveniently be studied in the environment where it is cultured. Moreover, this simplifies the study of the at least one ovum or embryo over time since the at least one ovum or embryo does not have to be taken out of the incubator each time it is to be analyzed.

Optionally, the method may further comprise transmitting the interference pattern from inside of the incubator to a processing unit outside of the incubator. Preferably, the transmitting includes transmitting the interference pattern wirelessly. In this way, the processing unit does not have to be located inside of the incubator. For example, the processing unit may be located on a remote server.

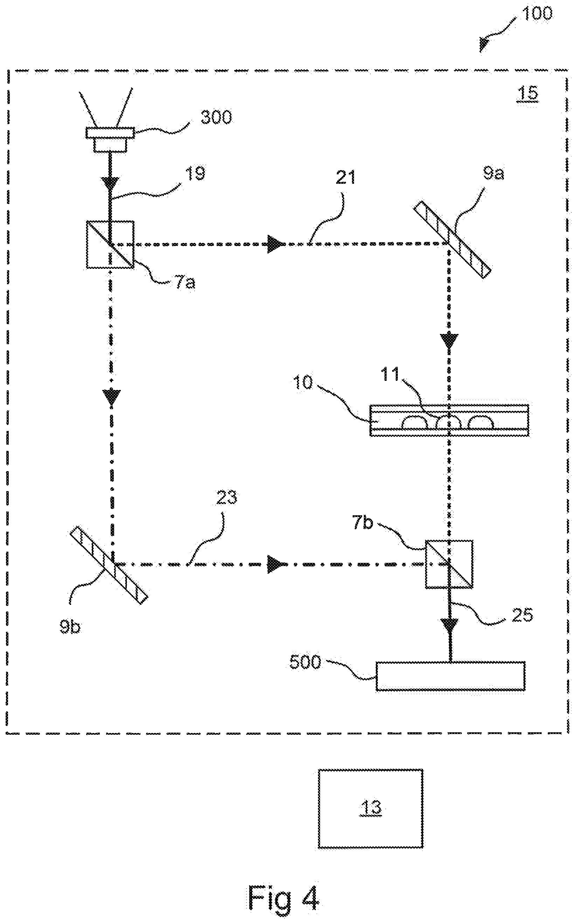

An apparatus according to an example not covered by the claims of the invention will now be described with reference to Fig 4. Fig 4 is a schematic illustration of an apparatus 100 for analyzing a sample 10. The apparatus 100 comprises at least one light source 300, a sensor 500, beam splitters 7a and 7b, reflecting surfaces 9a and 9b, and a processing unit 13. The beam splitters 7a and 7b, together with the reflecting surfaces 9a and 9b, act as means for exposing the sample to at least one object beam 21 and means for superposing the at least one object beam 21 with at least one reference beam 23.

The light source 300 is arranged to create at least one beam 19 of coherent light. The beam 19 of coherent light may for example be a laser beam which originates from any kind of laser source, such as a diode laser emitting light at a wavelength of 635 nm. Here, only one light source is illustrated. In general, however, the apparatus 100 may comprise several light sources 300 which may be used simultaneously. Preferably, if several light sources 300 are used, these create light of different wavelengths. This may be advantageous since the sample 10 may react differently to light of different wavelengths. Hence more information about the sample may be obtained by using a plurality of light sources 300 having different wavelengths.

The beam 19 of coherent light originating from the light source 300 is directed towards a beam splitter 7a. The beam splitter 7a divides the at least one beam 19 of coherent light into at least one object beam 21 and at least one reference beam 23. With this construction, the at least one object beam 21 and the at least one reference beam 23 become mutually coherent, implying that they have the same frequency and exhibit a constant phase relationship during the course of time. In case more than one light source 300 is present, the beam splitter 7a may divide each of the beams 19 originating from the light sources 300 into a mutually coherent object beam 21 and a reference beam 23.

When the apparatus 100 is in use, a sample 10 comprising at least one ovum or embryo 11 is arranged in the light path of the at least one object beam 21. For example, the reflecting surface 9a may be used to redirect the at least one object beam 21 such that the sample 10 is exposed to the at least one object beam 21. As the object beam 21 incides towards the sample 10, the object beam 21 will pass through the sample 10 and, in particular, through the at least one ovum or embryo 11. Since the object beam 21 passes through the sample 10, it will travel a longer optical path length compared to a beam, such as the reference beam 23, which does not pass through the sample, due to differences in refractive index. This will in turn lead to a phase shift between the object beam 21 and the reference beam 23. The optical path length is defined as the physical/geometrical thickness multiplied with the refractive index.

The reflecting surface 9b may be arranged to reflect and thereby redirect the at least one reference beam 23. Specifically, the reflecting surfaces 9a and 9b may be arranged such that the at least one object beam 21 and the at least one reference beam 23 are directed towards a means for superposing, here in the form of beam splitter 7b. The beam splitter 7b superposes the at least one object beam 21 and the at least one reference beam 23 and directs the superposed beam 25 towards the sensor 500.

The sensor 500 is arranged to detect an interference pattern arising from the object beam 21 and the reference beam 23. As the object beam 21 and the reference beam 23 are mutually coherent, they will generate an interference pattern at the sensor 500. In particular, since the at least one object beam 21 and the at least one reference beam 23 have traveled different optical path lengths due to the passage of the at least one object beam 21 through the sample 10, the interference pattern is indicative of the phase shift between the object beam 21 and the reference beam 23. The sensor 500 may for example be a digital sensor such as a CCD (Charge Coupled Device) or a CMOS (Complementary Metal Oxide Semiconductor) image sensor.

The sensor 500 may further be operatively connected to the processing unit 13 which may comprise software and/or hardware for carrying out any common reconstruction process. The reconstruction process may reconstruct phase and/or amplitude information from the interference pattern detected by the sensor 500. The reconstructed information may for example be used to obtain at least one analysis image of the studied at least one ovum or embryo 11. The information may for example also be used to determine the shape and optical density of the at least one ovum or embryo 11. Specifically, the processing unit 13 is arranged to determine at least one ovum or embryo quality showing parameter from the phase and/or amplitude information. In other words, the processing unit 13 may be arranged to carry out any data processing steps of the method according to embodiments of the invention.

Further, the apparatus 100 may comprise storage media or a memory (not shown). The storage media or memory may be operatively connected to the processing unit 13. Specifically, the storage media or memory may be arranged to store analysis images and time data pertaining to time-points at which changes were detected in the analysis images.

The apparatus 100 may be adapted to operate inside an incubator 15. With such an arrangement, the sample 10 does not have to be taken out of the incubator where it is cultured in order to be analyzed. As illustrated in Fig 4, the light source 300, the sensor 500, and the optical components, such as the beam splitters 7a and 7b and the reflecting surfaces 9a and 9b, are comprised inside of the incubator 15. The processing unit 13 may either be located inside the incubator 15 or outside of the incubator 15. In case the processing unit is located outside of the incubator 15, it is preferred if the processing unit may communicate wirelessly with the sensor 500. For example, the apparatus may comprise a transmitter (not shown) which is arranged to transmit information pertaining to an interference pattern from the sensor 500 to a receiver operatively connected to the processing unit. The transmitter may form part of a transceiver which is arranged to transmit signals to as well as receiving signals from the processing unit 13. Alternatively, the apparatus 100 may comprise an incubator 15. In this case, the incubator 15 is preferably formed integrally with the apparatus 100. Such an arrangement offers a very compact and flexible solution. For example, the apparatus 100, including the integrally formed incubator, may be put on a working desk and may be conveniently moved to another location if desired. Advantageously, by having an incubator 15 integrally formed with the apparatus 100, not all the components of the apparatus have to be arranged inside of the incubator. For example, some of the optical components, such as beam splitters 7a and 7b and reflecting surfaces 9a and 9b, which are sensitive to the conditions inside of the incubator may be arranged outside of the incubator. In one embodiment only the sample holder holding the sample 10 is located inside of the incubator 15.

The apparatus 100 may further comprise a controller (not shown) for controlling at least one of a humidity, a temperature and a carbon dioxide percentage inside of the incubator 15. In this way, optimal conditions for culture of the at least one ovum or embryo may be achieved. Cultured cells need certain tightly regulated parameters. First of all the cell culture medium needs to hold a pH of 7.4. As cell culture media most often are buffered with carbonate, this is achieved by supplying 5% CO₂ into the atmosphere, although an organic buffer such as HEPES can be used instead. In order to hinder the evaporation of the medium, the atmosphere should be water-saturated. For example, the humidity may be about or above 90-95%. Cultured cells preferably grow at 37 degrees C, although this is cell species dependent. E.g. mammalina cells prefer 37 degrees C, insect cells prefer 20 degrees C, while avian cells prefer 40-42 degrees C.

Further, different strategies may be used in order for the apparatus 100, and in particular the optical components of the apparatus 100, such as the beam splitters 7a and 7b and the reflecting surfaces 9a and 9b, to cope with the conditions inside of the incubator 15. According to one strategy, the apparatus 100 is arranged inside of the incubator 15 while the temperature inside of the incubator is essentially equal to room temperature. Then, the temperature inside of the incubator is increased at a low rate. In this way, condensation at the optical components of the apparatus 100 may be avoided since there will be no temperature difference between the optical components and the air inside of the incubator 15. According to another strategy, the optical components, such as beam splitters 7a and 7b and reflecting surfaces 9a and 9b may be arranged and/or treated to be able to cope with the conditions inside of the incubator 15. Specifically, the optical components may be treated to avoid condensation. In this way, the apparatus 100 may be arranged inside of the incubator 15 when the temperature inside of the incubator 15 is not essentially equal to room temperature. As a result, the above procedure of slowly increasing the temperature may be avoided.

As the skilled person readily understands, the example not covered by the claims described with reference to Fig 4 is just an illustrative and schematic example of a set-up. Many variations and modifications are possible. For example, there are many possible variations regarding the particular geometrical and optical set-up used in order to expose the sample to the at least one object beam, and to superpose the at least one object beam and the at least one reference beam.

### Detailed description of the drawings

In fig 1 two embryos (1) show the first sign of a successful fertilization, since more than one pronucleus (3) is visible in the single embryonic cell (2). The left embryo has two pronuclei (3), which is normal, and is therefore considered of high quality. However, the right one displays three pronuclei (3) and is therefore considered being of low quality. Nucleolar precursor bodies are visible as tiny spots within the pronuclei.

In fig 2 a zona pellucida (5) surrounds a polar body (4) and a single embryonic cell (2) of an embryo (1).

In fig 3 two embryos (1) comprising several cells (6) are visible. The upper embryo (1) is a morula having 8 cells (6). The upper embryo (1) comprises cells (6) having essentially the same size and is therefore considered of high quality. The lower embryo (1) is severely fragmented (has several cells and cell fragments (6) of different sizes) and is therefore considered being of low quality. The zona pellucida (5) of the embryos in fig 3 is weaker than for a zygote.

## Claims

1. Use of a method for analyzing a sample (10) comprising at least one ovum or embryo (1, 11), the method being solely based on digital holographic imaging, the method comprising the following steps:
a) creating at least one object beam (21) and at least one reference beam (23) of light, where said at least one object beam (21) and said at least one reference beam (23) are mutually coherent;
b) exposing said sample (10) to said at least one object beam (21);
c) superimposing said at least one object beam (21) that has passed through said sample (10) with said at least one reference beam (23) and thereby creating an interference pattern;
d) detecting said interference pattern, called hologram;
e) reconstructing phase and/or amplitude information of object wavefront from said interference pattern; and
f) constructing at least one ovum or embryo analysis image and determining at least one ovum or embryo quality showing parameter from said phase and/or amplitude information,
wherein the at least one ovum or embryo quality showing parameter embodies at least one of the parameters chosen from the group consisting of the radial thickness of the zona pellucida (5) of the at least one ovum or embryo (1, 11); the optical density of the zona pellucida (5) of the at least one ovum or embryo (1, 11); the dry mass, morphology or area of the at least one ovum or at least one cell (2) inside the at least one embryo; and the dry mass, morphology or area of at least one polar body (4);
and wherein step b) to d) are repeated at different time-points or continuously in order to reconstruct phase and/or amplitude information according to step e) and construct ovum or embryo analysis images according to step f) captured at said time-points or continuously.

2. Use according to claim 1, wherein at least the steps a)-d) are performed inside an incubator (15).

3. Use according to claim 2, further comprising transmitting said interference pattern from inside of said incubator (15) to a processing unit (13) outside of said incubator (15).

4. Use according to anyone of the preceding claims, wherein the sample (10) comprises more than one ovum (1, 11) and an ovum quality showing parameter is determined by comparing the dry mass, morphology or area of one ovum (1, 11) to at least one other; or the at least one embryo (1, 11) encapsulates more than one cell and an embryo quality showing parameter is determined by comparing the dry mass, morphology or area of one cell inside the at least one embryo (1, 11) to at least one other.

5. Use according to anyone of the preceding claims, wherein an ovum quality showing parameter is determined by counting the number of ova (1, 11) in the sample (10); or an embryo quality showing parameter is determined by counting the number of cells inside the at least one embryo (1, 11); or counting the number of pronuclei (3) inside the at least one ovum or embryo (1, 11).

6. Use according to anyone of the preceding claims, wherein the at least one ovum or embryo (1, 11) is at least one embryo encapsulating more than one cell and an embryo quality showing parameter is determined by determining the total dry mass of at least one defined space inside the at least one embryo (1, 11).

7. Use according to anyone of the preceding claims, wherein the at least one ovum or embryo quality showing parameter embodies the level of fragmentation and/or the degree of synchronization.

8. Use according to anyone of the preceding claims, wherein two or more wavelengths are used, the steps a) to e) are performed for each of the used wavelengths and the at least one ovum or embryo analysis image is constructed and the at least one ovum or embryo quality showing parameter is determined from the phase and/or amplitude information reconstructed using the two or more wavelengths.

9. Use according to claim 8, wherein the at least one ovum or embryo quality showing parameter embodies the volume of the at least one ovum or embryo (1, 11) or the volume of at least one cell inside the at least one embryo (1, 11) and/or the refractive index of at least one defined space inside the at least one ovum or embryo (1, 11).

10. Use according to anyone of the preceding claims, wherein the sample (10) comprising the at least one ovum or embryo (1, 11) is rotated and ovum or embryo analysis images are taken from different angles for the creation of a real three-dimensional reconstruction.

11. Use according to claim 10, wherein the ovum or embryo analysis images taken from different angles are combined by a tomographic algorithm for the creation of the real three-dimensional reconstruction.

12. Use according to any of claims 1-11, further comprising comparing an ovum or embryo analysis image captured at one of said time-points or continuously to an ovum or embryo analysis image captured at an earlier time point; detecting whether a change has occurred or not based on the result of the step of comparing; and, if a change has been detected, storing the time-point at which the change was detected.

## Patentansprüche

1. Verwendung eines Verfahrens zum Analysieren einer Probe (10), die mindestens eine Eizelle oder mindestens einen Embryo (1, 11) umfasst, wobei das Verfahren alleinig auf digitaler holographischer Bildgebung basiert, wobei das Verfahren die folgenden Schritte umfasst:
a) Erzeugen von mindestens einem Objektstrahl (21) und mindestens einem Referenzstrahl (23) von Licht, wobei der mindestens einen Objektstrahl (21) und der mindestens eine Referenzstrahl (23) gegenseitig kohärent sind;
b) Aussetzen der Probe (10) zu dem mindestens einen Objektstrahl (21);
c) Überlagern des mindestens einen Objektstrahls (21), der durch die Probe (10) gelaufen ist, mit dem mindestens einen Referenzstrahl (23) und dadurch Erzeugen eines Interferenzmusters;
d) Detektieren des Interferenzmusters, genannt Hologramm;
e) Rekonstruieren von Phasen- und/oder Amplitudeninformationen einer Objektwellenfront vom Interferenzmuster; und
f) Konstruieren von mindestens einem Eizellen- oder Embryonenanalysebild und Bestimmen von mindestens einem eizellen- oder embryonenqualitätsanzeigenden Parameter aus den Phasen- und/oder Amplitudeninformationen,
wobei der mindestens eine eizellen- oder embryonenqualitätsanzeigende Parameter mindestens einen der Parameter verkörpert, der aus der Gruppe bestehend aus der radialen Dicke der Zona Pellucida (5) der mindestens einen Eizelle oder des mindestens einen Embryonen (1, 11); der optischen Dicke der Zona Pellucida (5) der mindestens einen Eizelle oder des mindestens einen Embryonen (1, 11); der Trockenmasse, Morphologie oder Fläche der mindestens einen Eizelle oder von mindestens einer Zelle (2) innerhalb des mindestens einen Embryonen; und der Trockenmasse, Morphologie oder Fläche von mindestens einem Polkörper (4) ausgewählt wird; und wobei Schritte b) bis d) zu unterschiedlichen Zeitpunkten oder kontinuierlich wiederholt werden, um Phasen- und/oder Amplitudeninformationen gemäß Schritt e) zu rekonstruieren und Eizellen- oder Embryonenanalysebilder gemäß Schritt f), die zu den Zeitpunkten oder kontinuierlich erfasst werden, zu konstruieren.

2. Verwendung nach Anspruch 1, wobei zumindest die Schritte a)-d) innerhalb eines Inkubators (15) durchgeführt werden.

3. Verwendung nach Anspruch 2, ferner umfassend Übertragen des Interferenzmusters von innerhalb des Inkubators (15) zu einer Verarbeitungseinheit (13) außerhalb des Inkubators (15).

4. Verwendung nach einem der vorangegangenen Ansprüche, wobei die Probe (10) mehr als eine Eizelle (1, 11) umfasst und ein eizellenqualitätsanzeigender Parameter bestimmt wird, indem die Trockenmasse, Morphologie oder Fläche einer Eizelle (1, 11) mit mindestens einer anderen verglichen wird; oder der mindestens eine Embryo (1, 11) mehr als eine Zelle einkapselt und ein embryonenqualitätsanzeigender Parameter bestimmt wird, indem die Trockenmasse, Morphologie oder Fläche einer Zelle innerhalb des mindestens einen Embryonen (1, 11) mit mindestens einer anderen verglichen wird.

5. Verwendung nach einem der vorangegangenen Ansprüche, wobei ein eizellenqualitätsanzeigender Parameter bestimmt wird, indem die Anzahl von Eizellen (1, 11) in der Probe (10) gezählt wird; oder ein embryonenqualitätsanzeigender Parameter bestimmt wird, indem die Anzahl von Zellen innerhalb des mindestens einen Embryonen (1, 11) gezählt wird; oder Zählen der Anzahl von Vorkernen (3) innerhalb der mindestens einen Eizelle oder des mindestens einen Embryonen (1, 11).

6. Verwendung nach einem der vorangegangenen Ansprüche, wobei die mindestens eine Eizelle oder der mindestens eine Embryo (1, 11) mindestens ein Embryo ist, der mehr als eine Zelle einkapselt, und ein embryonenqualitätsanzeigender Parameter bestimmt wird, indem die Gesamttrockenmasse von mindestens einem definierten Raum innerhalb des mindestens einen Embryonen (1, 11) bestimmt wird.

7. Verwendung nach einem der vorangegangenen Ansprüche, wobei der mindestens eine eizellen- oder embryonenqualitätsanzeigende Parameter den Grad an Fragmentierung und/oder den Grad an Synchronisation verkörpert.

8. Verwendung nach einem der vorangegangenen Ansprüche, wobei zwei oder mehr Wellenlängen verwendet werden, die Schritte a) bis e) für jede der verwendeten Wellenlängen verwendet werden und das mindestens eine Eizellen- oder Embryonenanalysebild konstruiert wird und der mindestens eine eizellen- oder embryonenqualitätsanzeigende Parameter bestimmt wird aus den Phasen- und/oder Amplitudeninformationen, die unter Verwendung der zwei oder mehr Wellenlängen rekonstruiert werden.

9. Verwendung nach Anspruch 8, wobei der mindestens eine eizellen- oder embryonenqualitätsanzeigende Parameter das Volumen der mindestens einen Eizelle oder des mindestens einen Embryonen (1, 11) oder das Volumen von mindestens einer Zelle innerhalb des mindestens einen Embryonen (1, 11) und/oder den Brechungsindex von mindestens einem definierten Raum innerhalb der mindestens einen Eizelle oder des mindestens einen Embryonen (1, 11) verkörpert.

10. Verwendung nach einem der vorangegangenen Ansprüche, wobei die Probe (10), die die mindestens eine Eizelle oder den mindestens einen Embryonen (1, 11) umfasst, rotiert wird und Eizellen- oder Embryonenanalysebilder von verschiedenen Winkeln aus aufgenommen werden, um eine reale dreidimensionale Rekonstruktion zu erzeugen.

11. Verwendung nach Anspruch 10, wobei die Eizellen- oder Embryonenanalysebilder, die von verschiedenen Winkeln aus aufgenommen werden, durch einen tomografischen Algorithmus kombiniert werden, um die reale dreidimensionale Rekonstruktion zu erzeugen.

12. Verwendung nach einem der Ansprüche 1-11, ferner umfassend Vergleichen eines Eizellen- oder Embryonenanalysebildes, das zu einem der Zeitpunkte oder kontinuierlich erfasst wird, mit einem Eizellen- oder Embryonenanalysebild, das zu einem früheren Zeitpunkt erfasst wird; Detektieren, ob eine Änderung aufgetreten ist oder nicht, basierend auf dem Ergebnis des Schritts des Vergleichens; und, falls eine Änderung detektiert worden ist, Speichern des Zeitpunkts, zu dem die Änderung detektiert wurde.

## Revendications

1. Utilisation d'un procédé pour l'analyse d'un échantillon (10) comprenant au moins un ovule ou embryon (1, 11), le procédé étant seulement basé sur l'imagerie holographique numérique, le procédé comprenant les étapes suivantes :
a) créer au moins un faisceau objet (21) et au moins un faisceau de référence (23) de lumière, ledit au moins un faisceau objet (21) et ledit au moins un faisceau de référence (23) étant mutuellement cohérents ;
b) exposer ledit échantillon (10) audit au moins un faisceau objet (21) ;
c) superposer ledit au moins un faisceau objet (21) qui a traversé ledit échantillon (10) avec ledit au moins un faisceau de référence (23) et créer ainsi un motif d'interférence ;
d) détecter ledit motif d'interférence, appelé hologramme ;
e) reconstruire des informations de phase et/ou d'amplitude de front d'onde objet à partir dudit motif d'interférence ; et
f) construire au moins une image d'analyse d'ovule ou d'embryon et déterminer au moins un paramètre montrant la qualité d'ovule ou d'embryon à partir desdites informations de phase et/ou d'amplitude,
dans laquelle l'au moins un paramètre montrant la qualité d'ovule ou d'embryon représente au moins un des paramètres choisis dans le groupe constitué par l'épaisseur radiale de la zone pellucide (5) de l'au moins un ovule ou embryon (1, 11) ; la densité optique de la zone pellucide (5) de l'au moins un ovule ou embryon (1, 11) ; la masse sèche, la morphologie ou la surface de l'au moins un ovule ou d'au moins une cellule (2) à l'intérieur de l'au moins un embryon ; et la masse sèche, la morphologie ou la surface d'au moins un corps polaire (4) ;
et dans lequel les étapes b) à d) sont répétées à différents points temporels ou de façon continue afin de reconstruire des informations de phase et/ou d'amplitude selon l'étape e) et construire des images d'analyse d'ovule ou d'embryon selon l'étape f) capturées auxdits points temporels ou de façon continue.

2. Utilisation selon la revendication 1, dans laquelle au moins les étapes a)-d) sont effectuées à l'intérieur d'un incubateur (15).

3. Utilisation selon la revendication 2, comprenant en outre la transmission dudit motif d'interférence depuis l'intérieur dudit incubateur (15) à une unité de traitement (13) à l'extérieur dudit incubateur (15).

4. Utilisation selon l'une quelconque des revendications précédentes, dans laquelle l'échantillon (10) comprend plus d'un ovule (1, 11) et un paramètre montrant la qualité d'ovule est déterminé en comparant la masse sèche, la morphologie ou la surface d'un ovule (1, 11) à celle d'au moins un autre ; ou l'au moins un embryon (1, 11) renferme plus d'une cellule et un paramètre montrant la qualité d'embryon est déterminé en comparant la masse sèche, la morphologie ou la surface d'une cellule à l'intérieur de l'au moins un embryon (1, 11) à celle d'au moins un autre.

5. Utilisation selon l'une quelconque des revendications précédentes, dans laquelle un paramètre montrant la qualité d'ovule est déterminé en comptant le nombre d'ovules (1, 11) dans l'échantillon (10) ; ou un paramètre montrant la qualité d'embryon est déterminé en comptant le nombre de cellules à l'intérieur de l'au moins un embryon (1, 11) ; ou en comptant le nombre de pronucléi (3) à l'intérieur de l'au moins un ovule ou embryon (1, 11).

6. Utilisation selon l'une quelconque des revendications précédentes, dans laquelle l'au moins un ovule ou embryon (1, 11) est au moins un embryon renfermant plus d'une cellule et un paramètre montrant la qualité d'embryon est déterminé en déterminant la masse sèche totale d'au moins un espace défini à l'intérieur de l'au moins un embryon (1, 11).

7. Utilisation selon l'une quelconque des revendications précédentes, dans laquelle l'au moins un paramètre montrant la qualité d'ovule ou d'embryon représente le niveau de fragmentation et/ou le degré de synchronisation.

8. Utilisation selon l'une quelconque des revendications précédentes, dans laquelle au moins deux longueurs d'onde sont utilisées, les étapes a) à e) sont effectuées pour chacune des longueurs d'onde utilisées et l'au moins une image d'analyse d'ovule ou d'embryon est construite et l'au moins un paramètre montrant la qualité d'ovule ou d'embryon est déterminé à partir des informations de phase et/ou d'amplitude reconstruites en utilisant les au moins deux longueurs d'onde.

9. Utilisation selon la revendication 8, dans laquelle l'au moins un paramètre montrant la qualité d'ovule ou d'embryon représente le volume de l'au moins un ovule ou embryon (1, 11) ou le volume d'au moins une cellule à l'intérieur de l'au moins un embryon (1, 11) et/ou l'indice de réfraction d'au moins un espace défini à l'intérieur de l'au moins un ovule ou embryon (1, 11).

10. Utilisation selon l'une quelconque des revendications précédentes, dans laquelle l'échantillon (10) comprenant l'au moins un ovule ou embryon (1, 11) est tourné et des images d'analyse d'ovule ou d'embryon sont prises depuis différents angles pour la création d'une reconstruction tridimensionnelle réelle.

11. Utilisation selon la revendication 10, dans laquelle les images d'analyse d'ovule ou d'embryon prises depuis différents angles sont combinées par un algorithme tomographique pour la création de la reconstruction tridimensionnelle réelle.

12. Utilisation selon l'une quelconque des revendications 1 à 11, comprenant en outre la comparaison d'une image d'analyse d'ovule ou d'embryon capturée à un desdits points temporels ou de façon continue à une image d'analyse d'ovule ou d'embryon capturée à un point temporel antérieur ; la détection qu'un changement s'est produit ou non sur la base du résultat de l'étape de comparaison ; et, si un changement a été détecté, le stockage du point temporel auquel le changement a été détecté.
